# EUROPÄISCHE PATENTSCHRIFT

(11) **EP 2 185 208 B1**
(45) Veröffentlichungstag und Bekanntmachung des Hinweises auf die Patenterteilung: **18.01.2012**
(21) Anmeldenummer: 08801143.2
(22) Anmeldetag: 05.08.2008
(51) Int. Cl.: A61L 27/14, A61L 27/54, A61L 27/34, A61L 31/10, A61L 31/16

(54) **BRUST-IMPLANTAT MIT ANTIBAKTERIELLER WIRKUNG**
BREAST IMPLANT HAVING ANTIBACTERIAL EFFECT
IMPLANT MAMMAIRE À EFFET ANTIBACTÉRIEN

(30) Priorität: 21.08.2007 DE 102007039871
(43) Veröffentlichungstag der Anmeldung: 19.05.2010
(73) Patentinhaber: BioCer Entwicklungs-GmbH, 95447 Bayreuth (DE)
(72) Erfinder: KOKOTT, Andreas, 95138 Bad Steben (DE); HOFFMANN, Bettina, 96264 Altenkunstadt (DE); FELDMANN, Martina, 95488 Eckersdorf (DE); ZIEGLER, Günter, 90451 Nürnberg (DE); PRANTL, Lukas, 93047 Regensburg (DE); EISENMANN-KLEIN, Marita, 93080 Regensburg (DE)
(74) Vertreter: Donath, Dirk
(86) Internationale Anmeldenummer: PCT/DE2008/001310
(87) Internationale Veröffentlichungsnummer: WO 2009/024121

(56) Entgegenhaltungen:
- DE-A1- 10 243 132
- US-A- 5 344 451
- US-A1- 2007 160 641

## Beschreibung

Die Erfindung betrifft ein Brust-Implantat mit antibakterieller Wirkung im Bereich der Medizin.

Weichgewebe-Implantate werden zur Auffüllung von Gewebe eingesetzt.
Das wohl bekannteste Weichgewebe-Implantat ist das Brustimplantat, das zur Vergrößerung des Brustvolumens eingesetzt wird.

Brustimplantate bestehen in der Regel aus einer Polymerhülle, vorzugsweise Silikon. Um eine möglichst Gewebe-ähnliche Konsistenz zu erhalten, wird das Implantat mit Silikon (hoch kohäsiv) oder isotonischer Kochsalzlösung (NaCl) gefüllt.

Bei Brustimplantaten kann es nach der Implantation zu verschiedenen Komplikationen kommen, wozu auch die Infektion gehört.
Ein medizinisches Implantat wird bei seiner bestimmungsgemäßen Verwendung durch eine vorgesehene kleine, operativ hergestellte Körperöffnung an den Implantationsort gebracht. Dabei kommt das Implantat unweigerlich in Kontakt mit der Haut, welche die Öffnung umgibt.
In Folge der bakteriellen Kontamination des Implantates kann es postoperativ zu einer Infektion kommen (Vinh D. C. et al., Breast Implant Infection with Mycobacterium Fortuitum Group: Report of Case and Review, Journal of Infection 2006, Band 52, e63-e67).
Laut Literatur treten bei 9 % der Operationen Infektionen auf (Aesthetic Surg J 2005; Band 25, Seite 249-254; Antonio Roberto Basile, MD; Filipe Basile, MD; and Arthur Volpe Dangieri Basile: Late Infection Following Breast Augmentation With Textured Silicone Gel-Filled Implants). Ca. 2 % der Infektionen werden innerhalb der ersten 20 Tage nach der Operation beobachtet und ca. 3 % zwischen 20-288 Tagen (durchschnittlich nach 82 Tagen).

Bakterien haben die Fähigkeit, einen Biofilm auf Oberflächen aufzubauen und darin über einen längeren Zeitraum zu überleben. Biofilme schützen Bakterien auch vor Antibiotika. Ist ein Biofilm auf einer Implantat-Oberfläche vorhanden, so können zwar die Bakterien außerhalb des Biofilms mit Wirkstoffen bekämpft werden, jedoch kann es sein, dass erst nach Wochen, Monaten oder auch Jahren die Ausbreitung einer Infektion in dem mit dem Implantat versehenen Körper kommen kann.

Ein anderes Problem ist die zunehmend auftretende Resistenzbildung von Mikroorganismen gegenüber Antibiotika. Daher ist es sinnvoll, eine Implantatoberfläche mit einer antibakteriellen Beschichtung zu versehen.

Eine andere Komplikation, neben dem Auftreten von resestenten Keimen, ist das Auftreten einer Kapselfibrose, die im schlimmsten Fall zur Explantation der Brustimplantate führt, weil die Kontraktion der Bindegewebskapsel von tastbaren Verhärtungen, Brustdeformation bis zur Ruptur der Silikonhülle führen kann.
Ca. 20 % der Implantatträgerinnen leiden unter einer Kapselfibrose (Benediktsson K. und Perbeck L., Capsular contracture around salinefilled and textured subcutaneously-placed implants in irradiated and nonirradiated breast cancer patients: Five Years of Monitoring of a Prospective Trial, Journal of Plastic, Reconstructive & Aesthetic Surgery 2006; Band 59, Seite 27-34).

Die Ursache der Kapselfibrose ist noch unbekannt. Diskutiert wird u.a. eine bakterielle Besiedlung des Implantates (Virden C.P. et al., Subclinical Infection of the Silicone Breast Implant Surface as a Possible Cause of Capsel Contracture, Aesth. Plast. Surg., 16 (1992) 173; Handel N. et al., The Date of Breast Implants: A Critical Analysis of Complications and Out-Comes; Plast. Reconstr. Surg., 86 (1995) 1521; Dopke M.K. et al., Characterisation of Microbial Presence at the Surface of Silicone Mammary Implants, Ann. Plast. Surg., 34 (1995) 563; Prantl L. et al., Clinical and morphological conditions in capsular contracture formed around silicone breast implants, Plast. Reconstr. Surg., 120 (2007) 275-284).

Die häufigste Art von Weichgewebe-Implantaten wird als Brustimplantat zur Vergrößerung der Brust, seltener zum Aufbau nach Tumorentnahme eingesetzt. Das Implantat besteht in der Regel aus einer Hülle aus Silikon, seltener aus Polyurethan, gefüllt mit Silikon, isotonische Kochsalzlösung, Sojaöl (was schon vom Markt genommen wurde) oder anderen Flüssigkeiten wie Kollagenlösung (siehe DE 195 30 249 A1), um dem Implantat in Bezug auf die Konsistenz eine ähnliche Eigenschaft wie Brustgewebe zu verleihen.

Die maschinellen Herstellung von Brustimplantaten ist bspw. aus EP 1 432 562 B1) bekannt.
Die Anfertigung von bestimmten Geometrien ist in EP 1 267 725 B1 offenbart.

Ein Brustimplantat mit körpereigenen Zellen in einer Polymermatrix auf Hydrogelbasis wird in DE 695 34 083 T2 vorgestellt.

Der Einsatz von antibiotisch wirksamen Metallionen ist bereits seit geraumer Zeit bekannt.

Auch ist die Komplexierung von Metallionen schon lange bekannt. So werden beispielweise Calcium-Ionen durch Ethylendiamintetraacetat (EDTA) bei Gerinnungsuntersuchungen von Blut komplexiert. Neben solchen Molekülen wie EDTA wurden auch schon Komplexierungsgruppen-haltige Polymere bzw. Polysaccharide angewendet. Bei Polysacchariden wirken Carboxylgruppen und Aminogruppen komplexierend, wobei normalerweise Aminogruppenhaltige Polysaccharide überwiegend benutzt werden.

Neben technischen Anwendungen wurde auch schon die Anwendung von Metallionen-Polysaccharid-Komplexen in der Medizin beschrieben. Beispielsweise wird bei der Tumorbehandlung ein Chitosan-Kupfer-Komplex wegen seiner erhöhten Aktivität im Gegensatz zu unkomplexierten Kupferionen eingesetzt (Yong Zheng et al.; Preparation of Chitosan-Copper Complexes and their Antitumor Activity; Bioorganic & Medicinal Chemistry Letters 16 (2006) 4127-4129).

Bekannt ist auch, dass implantierbare Systeme aus Chitin und Chitosan durch Änderung des pH-Wertes gezielt Wirkstoffe abgeben können, was sich u.a. durch eine Art steuerbare Membran aus diesen Materialien realisieren lässt (Eugene Khor und Lee Yong Lim; Implantable Applications of Chitin and Chitosan; Biomaterials 24 (2003) 2339-2349.

Darüber hinaus sind auch diverse Beschichtungen von Implantaten bekannt, wobei diese Beschichtungen antibakterielle Wirkung haben können.
Bekannte Verfahren wie Änderung der Hydrophobie/Hydrophilie, Einsatz von glatten oder strukturierten Oberflächen wurden bei allen Implantaten bereits untersucht.

In WO 2004/096308 wird bspw. eine Metallbeschichtung auf der Außenseite der Silikonhülle von Brustimplantaten beschrieben. Beschichtungen mit Polysacchariden, wie bspw. Chitosan sind aus DE 197 24 869 A1 bekannt. Weitere Polysaccharidbeschichtungen werden bspw. in DE 44 44 445 C2 offenbart.

WO 03/060003 A1 beschreibt die Herstellung von Kunststoffartikeln (Thermoplaste) für Gebrauchsgegenstände durch Extrusion (Spritzgießen), wobei der Polymermasse ein antibakteriell wirkender Metall-Chitosan-Komplex zugesetzt wird.

Ein antibakterieller Film wurde durch Polysaccharidbeschichtung hergestellt, die Silber-Nanopartikel enthält (Jinhong Fu et al.; Construction of Antibacterial Multilayer Films Containing; Journal of Biomedical Materials Research Part A, Volume 79A, 3 (2006) 665-674).

Eine antibakterielle Wirkung kann auch bei Silikonimplantaten durch eine Erhöhung der Hydrophobizität eingestellt werden, wobei die antibakterielle Wirkung durch eine Oberflächen-Silanisierung bzw. Polysaccharidbeschichtung ohne Metallionen bzw. Wirkstoffe erreicht wird (H. Tang et al.; Effect of Surface Modification of Silicone on Staphylococcus Epidermidis Adhesion and Colonization; Colloids and Surfaces B: Biointerfaces 51 (2006) 16-24).

Ein völlig resorbierbares Implantat wird durch die Vernetzung von Chitosan mit Glutaraldehyd hergestellt. Durch das Einbringen von radioaktivem Jod kann eine lokale Bestrahlung bei Brachytherapien realisiert werden (Abdel Kareem Azab et al.; Crosslinked Chitosan Implants as Potential Degradable Devices for Brachytherapy: In Vitro and in Vivo Analysis; Journal of Controlled Release 111 (2006) 281-289).

Eine andere Art von Beschichtung wird im Patent DE 102 43 132 B4 offenbart. Hier werden Kupferionen aus einer kupferhaltigen Titandioxidschicht freigesetzt, die auf Implantate aufgebracht wird. Diese Schicht wird durch metallorganische Titanoxid - Precursoren erzeugt und bedarf zur Bildung eine thermische Umsetzung.

Bekannt sind auch Wirkstofffreisetzungssysteme bei Implantaten, die antibiotische Wirkstoffe freisetzen:
DE 103 32 072 B4 beschreibt bspw. ein individuell anpassbares Wirkstofffreisetzungssystem, wobei in einem implantierbaren Träger mehrere Öffnungen vorhanden sind, in die dann Wirkstoffe verschiedener Art untergebracht werden können. Die Wirkstoffe werden in diesem System lokal in einem kleinen Bereich freigesetzt. Dieses Implantat kann, mit Antibiotika gefüllt, z.B. nach Entnahme eines Fixateurexterne in die entzündete Öffnung eingeschraubt werden. Mit dieser Technik ist eine großflächige und gleichmäßige Abgabe von Wirkstoffen, z.B. die Herstellung einer antibakteriellen Oberfläche auf einem Brustimplantat, nicht realisierbar.

In DE 10 2005 002 703 A1 wird eine antibiotische Beschichtung von Implantaten beschrieben, bei der die Beschichtung aus mindestens einem gesättigten, organischen, hydrophoben, niedermolekularen Matrixbildner, der einen Schmelzpunkt im Temperaturbereich von 45°C bis 100°C hat, in dem ein niedermolekulares, hydrophobes Additiv gelöst ist, besteht und dass in dem Gemisch aus Matrixbildner und Additiv ein Antibiotikum/Antibiotikum suspendiert ist und/oder in dem ein mit dem Gemisch aus Matrixbildner und Additiv mischbares Antibiotikum / Antiobiotika gelöst ist.

DE 102 54 215 A1 offenbart ein chirurgisches, antimikrobiell wirkendes Implantat mit einer Grundstruktur und einer zumindest teilweisen Beschichtung. Das Implantat weist alpha-Hydroxycarbonsäure-Oligomere (vorzugsweise Milchsäure - Oligomere) auf und/oder lässt diese nach der Implantation als Abbauprodukt entstehen. Die Beschichtung enthält Polyolmonofettsäureester, vorzugsweise Glycerinmonofettsäureester.

In DE 101 14 364 A1 wird ein Verfahren zur Herstellung von antibiotischen Kompositen beschrieben, wobei ein plastisch verformbares Salz, das aus mindestens einer kationischen Komponente einer protonierten Antibiotika-Base aus den Gruppen der Aminoglykosid-Antibiotika, der Lincosamid-Antibiotika und der Tetracyclin-Antibiotika und aus mindestens einer anionischen Komponente aus der Gruppe der organischen Sulfate und/oder organischen Sulfonate und/oder Fettsäureresten aufgebaut ist, als Bindemittel zur Fixierung von anorganischen Kompositbestandteilen und/oder organischen Kompositbestandteilen und ggf. unter Beimischung von Wasser zur Formgebung der Komposite insbesondere durch Pressen, Strangpressen, Walzen, Kalandrieren und Mahlen verwendet wird.

DE 101 14 245 A1 offenbart die Herstellung und Verwendung einer Antibiotikum- / Antibiotika - Zubereitung für die Human- und Veterinärmedizin, zur Behandlung lokaler mikrobieller Infektionen im Hart- und Weichgewebe. Die erfindungsgemäße Herstellung einer Antibiotikum-/Antibiotika-Zubereitung erfolgt dadurch, dass Wasser, eine amphiphile Komponente eines Vertreters der Alkylsulfate, Arylsulfate, Alkylarylsulfate, Cycloalkylsulfate, Alkylcycloalkylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Arylsulfonate, Alkylarylsulfonate, Cycloalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate, eine oder mehrere antibiotische Komponenten aus der Gruppe der Aminoglykosid-Antibiotika, der Lincosamid-Antibiotika und Tetracyclin-Antibiotika, eine organische Hilfskomponente und/oder eine anorganische Hilfskomponente und ggf. mindestens eine biologisch aktive Hilfskomponente miteinander vermischt werden und zu Formkörpern, Granulaten, Pulvern, Folien, Vliesen und Fäden geformt werden.

DE 101 14 244 A1 offenbart eine Antibiotikum-/Antibiotika-Zubereitung für resorbierbare und nichtresorbierbare Implantate für die Human- und Veterinärmedizin, zur Behandlung lokaler mikrobieller Infektionen im Hart- und im Weichgewebe. Diese Antibiotikum-/Antibiotika-Zubereitung ist ein Gemisch, bestehend aus mindestens einer amphiphilen Komponente eines Vertreters der Alkylsulfate, Arylsulfate, Alkylarylsulfate, Cycloalkylsulfate, Alkylcycloalkylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Arylsulfonate, Alkylarylsulfonate, Cycloalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate sowie mindestens einer antibiotischen Komponente aus den Gruppen der Aminoglykosid-Antibiotika, der Lincosamid-Antibiotika oder Tetracyclin-Antibiotika und gegebenenfalls mindestens einer wasserfreien, organischen Hilfskomponente und gegebenenfalls mindestens einer anorganischen Hilfskomponente und gegebenenfalls mindestens einer biologisch aktiven Hilfskomponente. Die erfindungsgemäße Antibiotikum-/Antibiotika - Zubereitung weist eine verzögerte Wirkstofffreisetzung auf.

DE 699 35 677 T2 offenbart eine antibiotische hydrophile Beschichtung.

US 2003 / 0 203 003 A1 offenbart ein abbaubares Implantat aus Fasern. Diese Fasern sind mit Wirkstoffen gefüllt, wobei durch Weben zwei verschiedener Fasern verschiedene Wirkstoffe oder innerhalb einer Faser in verschiedenen Bereichen auch verschiedene Wirkstoffe abgegeben werden können. Bei dieser technischen Lösung kann die Abgabe es Wirkstoffes flächig sein.

US 2007 / 0 160 641 A1 offenbart einen Stent, der für eine medizinische Vorrichtung Verwendung findet, wobei ein Schichtaufbau mit 2 Bereichen verschiedener Freisetzungskinetiken oder mehrere Schichten vorgesehen sein können.

DE 102 43 132 B4 offenbart ein Verfahren zur Herstellung einer biokompatiblen metallionenhaltigen Titanoxid-Beschichtung auf einem Implantat, wobei die Metallionen unter physiologischen Bedingungen eluierbar und homogen in der Beschichtung verteilt sind und antibiotisch wirken können. Die TiO₂-Schicht wird gemäß diesem Verfahren bei einer Temperatur von 100 bis 1000°C aufgebracht, wobei eine innere Schicht aus Cu oder Ag bestehen kann, so dass verschiedene Freisetzungskinetiken der entsprechenden Ionen bestehen können.

Die Aufgabe der vorliegenden Erfindung ist es, ein Brustimplantat mit antibakterieller Wirkung anzugeben, das die Nachteile des Standes der Technik vermeidet, indem die Bakterienkontamination von Brustimplantaten in Folge der Anlagerung von Bakterien und die Bildung von Biofilmen verhindert bzw. reduziert wird.

Erfindungsgemäß wird die Aufgabe durch ein Brust-Implantat gemäß Anspruch 1 gelöst. Vorteilhafte Ausgestaltungen sind in den nachgeordneten Ansprüchen angegeben.

Das Wesen der Erfindung besteht darin, dass mittels des erfindungsgemäßen Brustimplantats, metallionen flächige in mindestens einem Kurzzeit- und mindestens einem Langzeitfreisetzungsareal auf dem Implantat gebunden und im implantierten Zustand des Implantats durch unterschiedliche Freisetzungsraten bzw. -mechanismen über verschiedene Zeiträume (Kurzzeit- und Langzeitfreisetzung) an die Umgebung freisetzbar sind. Dabei können ein oder mehrere Metallionen freigesetzt werden.

Dadurch erfolgt erfindungsgemäß über das gesamte Implantat eine flächige und nicht nur eine lokale Freisetzung der entsprechenden Wirkstoffkonzentration, so dass nur geringe Wirkstoffkonzentrationen nötig sind.
Darüber hinaus bewirkt die Kurzzeitfreisetzung antibiotisch wirkender Stoffe eine Verhinderung einer Primärbesiedlung und die Langzeitfreisetzung antibiotischer Stoffe die Bekämpfung der verbleibenden, überlebenden Bakterien, so dass die Biofilmbildung verhindert wird.

Durch die erfindungsgemäße Kombination von Kurzzeit- und Langzeitfreisetzung antibiotisch wirkender Stoffe wird bereits kurz nach Beginn der Freisetzung eine wirksame Konzentration (zwischen MIN und MAX) erreicht, die über einen längeren Zeitraum andauert. Zudem lässt sich durch Auswahl der Kurzzeitfreisetzung gewährleisten, dass eine maximale Konzentration (MAX) nicht überschritten.

Die unterschiedlichen Konzentrationen von Langzeit- bzw. Kurzzeitfreisetzung werden dabei über verschiedene Freisetzungsmechanismen (z.B. Adsorption-Desorption, Diffusion, Ionenaustausch, Matrixabbau u.a.) und/oder über verschiedene räumliche getrennte Anordnungen (z.B. Schichtaufbau u.a.) realisiert.

Gemäß der Erfindung kann bei der Freisetzung von Ionen zwischen verschiedenen Freisetzungskinetiken gewählt werden.

Besonders vorteilhaft ist dabei die antibiotische Wirkung von Metallionen.

Erfindungsgemäß ist hierbei, die nicht lokal in einem Bereich ausgeführte Freisetzung, sondern die flächige Freisetzung über die gesamte Implantatoberfläche. Eine lokale Freisetzung bei einer großen Implantatoberfläche würde dazu führen, dass lokal zu hohe Konzentrationen freigesetzt werden, die sich dann um das Implantat gleichmäßig verteilen müssten. Implantate können zwar gemäß dem Stand der Technik in Wirkstoffe bzw. Wirkstofflösungen getaucht werden, dabei findet zwar auch eine flächenhafte Freisetzung statt, die aber nicht zeitlich einstellbar ist.

Die Erfindung soll nachfolgend an Hand der Ausführungsbeispiele näher erläutert werden, ohne die Erfindung auf diese zu beschränken:

### 1. Ausführungsbeispiel (Schichtweiser Aufbau)

Ein Brust- Implantat mit Silikonhülle wird kovalent durch Umsetzung mit einem heterobifunktionellen Azid (photochemische Kopplung) und anschließender Kopplung eines Polysaccharides (Chitosan und/oder Heparin) beschichtet. Eine Kupferlösung auf wässriger Basis wird mit der Oberfläche in Kontakt gebracht und die Kupferionen dabei vom Polysaccharid komplexiert (Freisetzungssystem F1). Anschließend wird das Implantat in eine gesättigte Lösung von Kupferacetat (organisches Lösungsmittel, z.B. Aceton) getaucht. Das Lösungsmittel wirkt als Quellmittel für das Silikonmaterial und sorgt für ein Eindringen des Kupfersalzes in die Silikonoberfläche (Freisetzungssystem F2). Die Freisetzung von F1 erfolgt durch Ionenaustausch (Kurzzeitfreisetzung), während F2 durch Diffusion von Kupferionen aus dem Silikon erfolgt (Langzeitfreisetzung).

### 2. Ausführungsbeispiel

Die Oberfläche eines Silikonimplantates wird durch eine Acrylsäure-Pfropfpolymerisation carboxyliert. Anschließend wird das Implantat in eine gesättigte Lösung von Kupferacetat in ein organisches Lösungsmittel (z.B. Aceton) getaucht. Das Lösungsmittel wirkt als Quellmittel für das Silikonmaterial und sorgt für ein Eindringen des Kupfersalzes in die Silikonoberfläche (F2). Anschließend wird das Implantat in eine wässerige Kupferionen - haltige Lösung getaucht, um die Kupferionen an den Carboxylgruppen zu komplexieren.
Die Freisetzung von F1 erfolgt durch Ionenaustausch an den Carboxylgruppen (Kurzzeitfreisetzung), während F2 durch Diffusion von Kupferionen aus dem Silikon erfolgt (Langzeitfreisetzung).

## Patentansprüche

1. Brust- Implantat mit einer Freisetzung von Metallionen bei dem ein oder mehrere Ionen flächig in mindestens einem Kurzzeit- und mindestens einem Langzeitfreisetzungsareal auf dem Implantat gebunden und im implantierten Zustand des Implantats freisetzbar sind, wobei die Freisetzung durch mindestens zwei verschiedene Freisetzungsmechanismen erfolgt, **dadurch gekennzeichnet, dass** zumindest je ein Freisetzungsmechanismus im Bereich des Kurzzeit- und des Langzeitfreisetzungsareals angeordnet ist, wobei der eine Freisetzungsmechanismus eine Diffusion der Metallionen und der andere Freisetzungsmechanismus Ionenaustausch ist, und sich die Freisetzungsareale über das gesamte Implantat erstrecken.

2. Brust- Implantat mit einer Freisetzung von Metallionen gemäß Anspruch 1, **dadurch gekennzeichnet, dass** die Freisetzung aus einer Matrix aus Polysacchariden, Polysaccharidderivaten, Polymeren, Copolymeren oder Polymer bzw. Co- Polymerblends bzw. aus Mischungen dieser erfolgt.

3. Brust- Implantat mit einer Freisetzung von Metallionen gemäß Anspruch 1 oder 2, **dadurch gekennzeichnet, dass** das Implantat aus abbaubaren oder nicht abbaubaren Materialien besteht.

4. Brust- Implantat gemäß Anspruch 1, 2 oder 3 zur Verwendung als nichtabbaubares Silikonimplantat oder Expanderprothese.

## Claims

1. Breast implant with a release of metal ions in which one or more substances are bound on the implant in a two-dimensional manner in at least one short-time and at least one long-time release region and said substances can be released in the implanted condition of the implant, wherein the release is achieved by at least two release mechanisms with at least one release mechanism being arranged in the short-time release region and at least one being arranged in the long-time release region, one release mechanism is a diffusion of the metal ions and the other release mechanism is an ion exchange, and the release regions extend over the whole implant.

2. Breast implant with a release of metal ions according to claim 1, wherein said metal ions are released from a matrix consisting of polysaccharides, polysaccharide derivatives, polymers, copolymers or polymer or copolymer blends or of the mixtures of them.

3. Breast implant with a release of metal ions according to claim 1 or 2, wherein the implant consists of degradable or non-degradable materials.

4. Breast implant according to claim 1, 2 or 3 for the use as a non-degradable silicone implant or expander prosthesis.

## Revendications

1. Implant mammaire avec un dégagement des ions métalliques sur lequel un ou plusieurs ions sont liés en surface dans au moins un terrain de dégagement de courte durée et dans au moins un terrain de dégagement de longue durée et peuvent être dégagés en état implanté de l'implant, le dégagement étant effectué à l'aide d'au moins deux mécanismes de dégagement différents, est **caractérisé en ce qu'**au moins un mécanisme de dégagement est respectivement positionné dans la zone du terrain de dégagement de courte durée et du terrain de dégagement de longue durée, une mécanisme de dégagement étant une diffusion des ions métallique et l'autre mécanisme de dégagement étant un échange d'ions, et les terrains de dégagement s'étendent à l'implant entier.

2. Implant mammaire avec un dégagement des ions métalliques suivant la revendication 1 est **caractérisé en ce que** le dégagement s'effectue à partir d'une matrice formée des polysaccharides, dérivés de polysaccharide, polymères, co-polymères ou des mixtures de polymères et/ou co-polymères et/ou de leurs mixtures.

3. Implant mammaire avec un dégagement des ions métalliques suivant la revendication 1 ou 2 est **caractérisé en ce que** l'implant se compose des matières dégradables ou non dégradables.

4. Implant mammaire suivant la revendication 1, 2 ou 3 pour être utilisé comme implant de silicone non dégradable ou prothèse d'expansion.
